# EUROPEAN PATENT APPLICATION

(11) **EP 1 321 515 A1**
(43) Date of publication of application: **25.06.2003**
(21) Application number: 01130596.8
(22) Date of filing: 21.12.2001
(51) Int. Cl.: C12N 5/06, C12N 5/00

(54) **Method of culturing cells**

(71) Applicant: Ingenium Pharmaceuticals AG, 82152 Martinsried (DE)
(72) Inventor: Runge, Dieter, 81245 München (DE)
(74) Representative: Akers, Noel James

(57) **Abstract**

This invention relates to a method for culturing cells comprising providing cells to be cultured and contacting the cells with a serum-free culture medium in the presence of a protein matrix, wherein the cells are cultured in the absence of serum. The invention further relates to cells obtainable by this method and a serum-free cell culture medium used in conjunction with a protein matrix.

## Description

The present relates to the field of *in vitro* cell culture, in particular to a method for culturing cells comprising providing cells to be cultured and contacting the cells with a serum-free culture medium in the presence of a protein matrix wherein the cells are subsequently cultured in the absence of serum supplement. The invention further relates to cells obtainable by this method and a chemically defined, serum-free cell culture medium comprising a protein matrix.

Generally, *in vitro* cell culturing is performed in the presence of serum. Serum is a complex mixture of serum proteins, immunoglobulins, cytokines, growth factors, hormones etc. Therefore, it serves as a universal supplement for the vast majority of cell cultures including cell lines and primary cells. Serum for use in cell culture applications is commercially available. It is usually of bovine origin, foetal bovine serum (FBS), also known as foetal calf serum (FCS), being the most common serum supplement.

However, serum composition varies with regard to origin, feeding situation and harvesting conditions. These differences make it necessary that every batch has to be tested for its suitability in cell culture.

Furthermore, since serum is a biological material, it has to be assayed for viral and bacterial contaminations, which is labour and cost intensive. In addition, import restrictions due to TSE (transmissible spongiform encephalopathy) have impaired free transport and shipment of biological material, cells and cell lines between laboratories in different countries recently.

The complex nature of serum which contains a myriad of undefined components has also presented complications in many areas of study, especially in hormonal or nutritional studies. This is due to the fact that the serum contains many non-defined constituents which lead to the generation of varying results depending on the batch of serum used. Some serum components may even interfere directly with the measurement of the substances to be analysed in a cell culture. Thus, the reproducibility of cell based assays used for the validation of drugs should be improved when serum-free media are used.

Culturing the cells in a serum-free medium would allow to investigate the effects of many cellular processes which are affected by the serum components. Studies of particular interest include the study of the effects of hormones, growth factors, and other gene regulators on protein expression, signal transduction pathways, and drug metabolism. Analysis of cell culture supernatants or purification of secreted proteins by means of HPLC, FPLC or other methods could also be greatly improved and simplified if conducted in a serum-free or chemically defined environment, since there would be a lower background unrelated to cell metabolism.

Liver and renal cells, and hepatocytes in particular are useful for assays to monitor liver and kidney specific functions in man. These cells could form the basis of promising *in-vitro* assay systems.

Hepatocytes are characterised by numerous physiological and patho-physiological functions including the metabolism of carbohydrates, amino acids, fatty acids and steroids, the synthesis of serum proteins, response to inflammatory stimuli, and the biotransformation of xenobiotics (drugs, food additives, environmental pollutants).

However, there are species specific differences in the regulation of protein expression, biotransformation of xenobiotics, and enzymatic reactions. Hence, hepatocyte cultures of human origin offer the most promising *in-vitro* assay system to follow or analyse hepatocyte specific biotransformation of xenobiotics and drug metabolism in man (Moshage and Yap 1992). Unfortunately, due to a shortage of organ donors, human hepatocytes that are not used for organ or cell transplantation are very rare.

Various extra-cellular support matrices like collagen, fibronectin, laminin or more complex and undefined bio-matrices like EHS gel (Matrigel™, Becton, Dickinson and Company, Franklin Lakes NJ USA) may serve as substrata for epithelial cells. The use of collagen (Michalopoulos et al., 1978; Sattler et al., 1978; Strom and Michalopoulos, 1982), laminin and fibronectin (Bissel et al., 1986, 1987) as two-dimensional matrices for the culture of primary rat hepatocytes has been introduced several years ago. It was demonstrated that protein matrices used as attachment surface instead of conventional plastic culture-ware have a beneficiary effect for attachment, differentiation status, and proliferative capacity of isolated primary cells.

USP 4,642,292 discloses a method for the isolation of a biomatrix containing a significant portion of the extra cellular matrix, i.e. basement membrane components and components of the ground substance. This biomatrix was tested on primary rat hepatocytes in cell culture medium supplemented with FBS.

USP 4,829,000 (EP 0 218 065) discloses a biologically active basement membrane composition (matrigel) which is capable of forming a three-dimensional matrix. Such matrix comprises many components of the basement membrane such as collagen, in particular type IV collagen, the glycoproteins laminin and entactin, nidogen and heparan sulfate proteoglycans, which can interact to form a gel. These matrigel compositions were tested on B16C3 melanoma cells and various other tumour cells in medium supplemented with FBS.

A further improvement was the establishment of three-dimensional culture conditions that allowed culture of primary hepatocytes in collagen gel cylinders or sandwiches (Nyberg et al., 1992, Bader et al., 1992, Michalopoulos et al., 1993). Rodent hepatocytes seem to maintain their highest degree of differentiation in the presence of the complex EHS gel matrix (Silva et al., 1998), which like serum contains a myriad of undefined components.

Recently, it has been shown that primary human hepatocytes maintained liver specific functions for some weeks when cultured in chemically defined medium on fibronectin or collagen coated plates (Ferrini et al., 1997, Runge et al., 2000a, 2000b). However, the cells cannot be sub-cultured effectively. Ferrini et al. disclose a serum-free culture of primary hepatocytes on type I collagen coated culture dishes. The disadvantage of their system is that the cells have to be incubated for at least four hours after initial plating to allow for attachment of the cells to the surface of the culture dish.

Primary cells have been cultured in the absence of serum on collagen coated culture dishes. WO 90/10060 discloses a cell culture method for primary primate hepatocytes in serum-free medium on collagen coated culture dishes. However, in order for the cells to attach to the dish, they were first incubated in 5% FBS for three hours. After that, the cells were maintained serum-free for 21-28 days after which period they started to degenerate, losing their hepatocyte morphology.

WO 92/15700 discloses a method for a native-state histoculturing system for skin biopsies grown on an extra cellular support matrix, e.g. a collagen containing gel. Although serum-free media are mentioned as being well known in the art, there is no disclosure of the cell culture having been performed using serum-free medium.

EP 0 684 309 disclosed methods for preparing dried collagen films for cell culture. Undifferentiated CACO-2 cells were cultured in serum-free DMEM supplemented with MITO+ serum extender in culture dishes coated with collagen. The cells were cultured only for a period of up to 14 days by which time they had differentiated.

The sustained serum-free culturing of cell lines without loss of healthy morphology or loss of differentiation has proven to be impossible. HuH7 hepatoma cells could be cultured serum-free for a few days, but had to be re-fed with serum after every cell propagation and on a regular basis. They also tended to display unhealthy morphologies (Yoo et al., 1995). Another report showed that HuH7 hepatoma cells could be propagated in serum-free medium on conventional plastic dishes but they displayed a very slow growth rate (Nakabayashi et al., 1982, 1984). These cells maintained hepatocyte specific functions, but failed to attach to the surface of the dish and died within a few days when plated at low densities, unless cell free conditioned medium collected from semi-confluent populations of HuH7 cells was added at a final concentration of 50%. Furthermore, these cells grew at a very slow rate, with a time between passages of 2 weeks or longer (Nakabayashi et al., 1984).

Another commonly used human hepatoma cell line is HepG2 (Aden et al., 1979, Knowles et al., 1980). In general, HepG2 cells are cultured in the presence of bovine or equine serum, very few short-term culture systems in the absence of serum have been described (Adeli and Sinkevitch, 1990; Tecce and Terrana, 1988). Adeli and Sinkevitch only cultured the cells serum-free for a maximum period of twelve days.

WO 98/51785 discloses a method for co-culturing cells including cell lines, in which first, a first cell type is contacted with a collagen coated substrate in a first medium and next, a second cell type is contacted with the substrate to which the first cell type has attached, in the presence of a second medium. One of these may be serum-free. The attachment medium contains serum and may additionally contain attachment factors such as antibodies or fibronectin. There is no disclosure of any long-term culture.

EP 0 351 458 discloses a serum-free culture of HepG2 cells, i.e. a hepatocyte cell line. This cell line was cultured in RPMI 1640 medium free of serum and maintained for about three weeks. There is no disclosure of any attachment factors or any long-term culture in serum-free medium.

Although primary cells have been propagated for a limited period of time on a protein matrix in serum-free medium, this has been more difficult to achieve with cell lines. Differentiated cells such as differentiated hepatocyte or hepatoma cell lines, e.g. HUH7 and HepG2 are difficult to maintain in a differentiated state.

The present invention provides a method for *in vitro* culture of cells and in particular cell lines in a serum-free medium which would make it possible to maintain the cells in a healthy and/or differentiated state for an extended period of time.

The cells that can be cultured according to the present invention are eukaryotic cells, in particular vertebrate cells, mammalian cells being preferred. Preferably, human cells are cultured according to the invention. Preferably, the cell types used in this method are cell types that are able to attach to a culture flask or dish, preferably epithelial cells. Preferred cells are those of renal or kidney origin, in particular hepatocytes and hepatoma cells.

The method according to the invention is particularly suitable for the cell culture of cell lines. Preferred cell lines are those of renal or kidney origin, in particular hepatocytes and hepatoma cells.

Most particularly, the invention relates to techniques by which human epithelial cells or cell lines, preferably of liver or kidney origin, can be cultured in serum-free medium in the presence of a protein matrix, in particular COS cells, HuH7 cells and HepG2 cells.

The method according to the present invention allows cell lines, in particular differentiated cell lines to be propagated in a serum-free medium without the cells losing their differentiated state and morphology. The cells can be cultured over an extended period of time without a substantial number of cells detaching from the culture receptacle surface or displaying any unhealthy features. An extended period of time is a period longer than 10 days. The method according to the invention allows cells to be cultured under the conditions stated herein for a period of more than 10 days, and preferably (in increasing order of preference): more than 21 days; more than 30 days; more than 45 days; more than 60 days; more than 90 days and, most preferably more than 120 days without the cells losing healthy morphology. With the method of the invention, cells can be maintained serum-free for three months and longer.

Another advantage of this method is that differentiated cells so cultured remain in the differentiated state for a prolonged period of time.

Unexpectedly, the present inventors found that the combination of the beneficiary effects of protein-matrices for cell attachment with a chemically defined medium makes it possible to culture differentiated human hepatoma cells without losing hepatocyte-specific functions.

The present invention provides a method for culturing cells of a cell line, comprising providing cells to be cultured, contacting said cells with a serum-free culture medium in the presence of a protein matrix, wherein the culture medium is not supplemented with serum.

Preferably, the cells are cultured under chemically defined conditions using chemically defined media. The term "chemically defined" for the purposes of this invention means that the medium contains no non-defined components such as are present in serum or other biological supplements.

In a preferred embodiment, the method of the invention is used for culturing cells, wherein the cells are cultured such that they attach and grow in the absence of serum supplement.

Unexpectedly, it was found that it was possible to culture eukaryotic cell lines which are capable of attachment to a surface for an extended period of time even when the cells were attached in the absence of serum and subsequently cultured in the absence of serum and in the presence of a protein matrix.

Using the method according to the present invention, these cells can be cultured and propagated for several weeks in the absence of serum. Two-dimensional gel electrophoretic analysis, metabolic activity assays and Western blots were performed to characterise the cells (see Examples). Hepatocyte specific function/gene expression is maintained in the absence of serum for several days and even after several passages.

It has been shown that culturing primary human hepatocytes in serum-free medium requires the presence of growth factors such as hepatocyte growth factor (HGF) or epidermal growth factor (EGF). Primary cells cannot be cultured without these growth factors (Runge et al. 2000a, 2000b).

Unexpectedly, it was found that in serum-free cultures of cell lines, in particular HUH7 and HepG2, the addition of HGF and EGF has a detrimental effect on cells from cell lines leading to cell death after few passages. Therefore, the method according to the invention is preferably carried out without supplementing the medium with EGF or HGF. Preferably, the medium is not supplemented with any growth factors.

In the method according to the invention, the cells are cultured in the presence of protein matrix. Such a protein matrix may be added to the media, or it may be coated onto the culture receptacle prior to adding the medium and the cells.

The protein matrix suitable for the purposes of this invention may be any component or mixture of components taken from basement membranes. Suitable protein matrix examples include: collagen, in particular type I or IV collagen, rat tail collagen, laminin, entactin, nidogen and heparin sulphate proteoglycans or other extracellular matrix proteins. Protein matrices in the form of a gel have been known for some time, for example, Matrigel™ or EHS gel, available fromcommercial sources. Matrigel™ is derived from the Engelbreth-Holm-Swarm (EHS) mouse sarcoma. The preferred protein matrices are collagen, laminin and fibronectins or combinations thereof. The protein matrix may also comprise other attachment factors such as antibodies or other factors that allow and/or facilitate the attachment of cells to the surface of a culture receptacle. It is preferred to use a protein matrix which does not add any non-defined substances to the culture conditions so that preferably, chemically defined conditions can be maintained.

In a preferred embodiment of the present invention, the protein matrix substrate is coated onto the culture dish or flask to be used for the cell culture method according to the present invention. Culture dishes and flasks coated with various protein matrix substances are commercially available. In a further preferred embodiment, culture flasks or dishes are coated with rat tail collagen by UV-cross-linking overnight under sterile conditions. This can be performed using any suitable method known in the prior art. However, it is also possible to add a suitable protein matrix to the serum-free medium to be used for cell culture.

Choosing the conditions for seeding and incubating is within the skill of a person skilled in the art. The preferred cell culture conditions are as follows. The cells are seeded at 1-2 x 10⁶ viable cells per three 75 ml flasks or per three 10 cm dishes. The cells are incubated at 37°C in a preferably humidified CO₂ atmosphere of about 5 %. The culture medium is renewed every 1 - 3 days and the cells are re-plated once they reach 80-100% confluence.

Cells maintained by the method according to the invention can be propagated for several months without degenerating or losing their differentiation characteristics.

Another aspect of the present invention is a cell culture system for *in vitro* culturing of cells of a cell line, comprising cells to be cultured, a suitable cell culture receptacle, a culture medium and a protein matrix substrate, wherein the serum-free medium is not supplemented with serum. Preferably, the medium is not supplemented with growth factors such as EGF or HGF.

According to a preferred embodiment, the cell culture receptacle is coated with the protein matrix. The protein matrix can be any of the above mentioned components.

Preferably the system comprises cells which have been cultured according to the cell culture method of the present invention.

Another aspect of the present invention relates to a cell culture kit for culturing differentiated cells of a cell line, comprising a serum-free cell culture medium and optionally further cell culture supplements, a cell culture receptacle and a protein matrix, wherein the serum-free medium is not supplemented with serum, and wherein the protein matrix is dissolved or suspended in the cell culture medium. The protein matrix can be any of the above mentioned components.

Another aspect of the present invention relates to a cell culture kit for the cell culture of cells of a cell line, comprising a serum-free cell culture medium and optionally further cell culture supplements, a cell culture receptacle and a protein matrix, wherein the serum-free medium is not supplemented with serum, EGF or HGF. Preferably, the cell culture receptacle is coated with the protein matrix which may be any of the above mentioned components.

According to a further aspect of the invention, a serum-free cell culture medium is provided which comprises a protein matrix dissolved or suspended in the medium.

The protein matrix is preferably selected from collagen, in particular type I or type IV collagen, rat tail collagen, laminin, EHS gel, entactin, nidogen, heparin sulphate proteoglycans and fibronectins and other extra-cellular protein matrix components or mixtures thereof. The matrix can be a biomatrix consisting of a number of the above components such as matrigel. Preferably, the matrix is a collagen matrix. Preferably, the collagen matrix is present at a concentration ranging from 0.005, preferably 0.01, preferably 0.015 to 0.025, preferably 0.05 mg/m², more preferably at 0.02 mg/m².

The medium is made up of a suitable basic medium such a minimal essential medium (MEM; Eagle 1959) or Dulbecco's Eagle medium (DMEM; Dulbecco & Freeman, 1959) or RPMI (e.g. RPMI 1640; Moore et al., 1967) or mixtures thereof. The skilled person is aware of various basic media. In a preferred embodiment, the basic medium consists of one part MEM and one part DMEM.

Preferably, the medium is supplemented with glutamine at a suitable concentration, such as from 0.1, preferably 0.2, preferably 0.3, to 0.6, preferably 0.8, preferably 1, preferably 2 mM, more preferably at 0.5 mM. Another preferred supplement is albumin, preferably at a concentration of from 0.5, preferably 0.75, preferably 0.8 to 1.2, preferably 1.5, preferably 2 g/l, more preferably at 1 g/l.

In further preferred embodiments, the basic medium is further supplemented with supplements selected from nutrients, amino acids, buffers, sugars, salts, trace elements, glucocorticoids, steroid hormones and differentiation factors.

Preferably, additional amino acids are selected from proline and ornithine. Proline is added preferably at a concentration ranging from 0.05 mM, preferably 0.08 mM, preferably 0.1 mM to 0.15 mM, preferably 0.2 mM, preferably 0.5 mM, more preferably at 0.13 mM. Ornithine is added at concentrations ranging from 0.05, preferably 0.1, preferably 0.2 to 0.5, preferably 0.7, preferably 0.8 mM, more preferably at 0.3 mM.

A suitable buffer is for example Hepes which is added preferably at concentration ranging from 1, preferably 2.5 to 7.5, preferably 10 mM, more preferably at 5 mM.

Galactose can be chosen as a suitable sugar supplement and added at concentrations ranging from 0.5, preferably 1.5, preferably 2.5, preferably 5 mM to 7.5, preferably 10 mM, more preferably at 5.5 mM.

Preferably, salts and/or trace elements are selected from selenium, Zn²⁺, Cu²⁺ and Mn²⁺. Selenium is preferably added in the form of selenite at 0,5 to 10 ng/ml, preferably 1 to 5 ng/ml, and most preferably as Na selenite at 2.5 ng/ml. Zinc, copper and manganese are preferably added as ZnCl₂, CuSO₄, ZnSO₄, MnSO₄ and added at concentrations ranging from 0.002, preferably 0.01 to 0.2, preferably 0.5 mg/ml.

In a preferred embodiment, the medium is supplemented with cell type specific differentiation factors. In the case of differentiated hepatocyte cells, these are preferably selected from proline, transferrin and insulin. Further optional supplements are selected from selenium and dexamethasone. Selenium is preferably added in the form of Na-selenite at concentrations in the range of 1, preferably 1.75, preferably 2 ng/ml to 2.75, preferably 3, preferably 4 ng/ml, more preferably at 2.5 ng/ml. Dexamethasone is preferably added at concentrations ranging from 10, preferably 20, preferably 30 nM to 60, preferably 80, preferably 100 nM, preferably at 50 nM. It is also possible and preferred to supplement with a mixture of insulin/transferrin/selenium. Insulin is preferably added at concentrations ranging from 1, preferably 2, preferably 2.5 µg/ml to 3 preferably 4, preferably 5 µg/ml.

The medium may be further supplemented with suitable antibiotics, antimycotics and/or antifungal agents, preferably selected from gentamycin, penicillin, streptomycin, anti-PPLO, linocin and fungizone.

Preferably, the medium is not supplemented with EGF or HGF. More preferably, the medium is not supplemented with any growth factors.

When hepatocyte cell lines are to be cultured, the most preferred liquid medium to be used has the following composition: 2 parts minimal essential medium with non-essential amino acids (MEM), 1 part Dulbecco's modified Eagle medium (DMEM) and 1 part Dulbecco's modified Eagle medium (DMEM) supplemented with 0.2 to 4 g/l, preferably 0.5 to 2 g/l, most preferably 1 g/l albumin; 1 to 20 mM, preferably 2 to 10 mM, most preferably 5.5 mM galactose, 0.05 to 2 mM, preferably 0.1 to 1 mM, most preferably 0.5 mM glutamine; 0.02 to 2 mM, preferably 0.05 to 1 mM, most preferably 0.3 mM ornithine; 0.02 to 1 mM, preferably 0.05 to 0.5 mM, most preferably 0.13 mM proline; 0.5 to 20 mM, preferably 1 to 10, most preferably 5 mM Hepes; 0,5 to 10 ng/ml, preferably 1 to 5 ng/ml selenite ion, more preferably as Na selenite, most preferably as Na selenite at 2.5 ng/ml; 0.5 to 10 µg/ml, preferably 1 to 5 µg/ml, most preferably 2.5 µg/ml transferrin; 0.001 to 0.3 mg/l Cu²⁺ ion, preferably as 0.005 to 1.0 mg/l CuSO₄.5H₂O, most preferably as 0.01 to 0.5 mg/l CuSO₄.5H₂O; 0.001 to 0.01 mg/l Mn²⁺ ion, preferably as 0.002 to 0.04 mg/l MnSO₄, most preferably as 0.005 to 0.02 mg/l MnSO₄; 0.02 to 1 mg/l Zn²⁺ ion, preferably as 0.05 to 1.5 mg/l ZnCl₂ plus 0.05 to 1.5 mg/l ZnSO₄.7H₂O, more preferably as 0.1 to 0.4 mg/l ZnCl₂ plus 0.1 to 0.5 mg/l ZnSO₄.7H₂O, and most preferably as 0.272 mg/l ZnCl₂ plus 0.375 mg/l ZnSO₄.7H₂O; 0.5 to 10 µg/ml, preferably 1 to 5 µg/ml, most preferably 2.75 µg/ml insulin; 5 to 200 nM, preferably 10 to 100 nM, most preferably 50 nM dexamethasone; 5 to 100 µg/ml, preferably 10 to 50 µg/ml, most preferably 25 µg/ml gentamycin; 20 to 400 U/ml, preferably 80 to 200 U/ml, most preferably 100 U/ml penicillin; and 25 to 400 µg/ml, preferably 50 to 200 µg/ml, most preferably 100 µg/ml streptomycin, and wherein the composition contains no animal serum. The medium for culturing cell lines comprises the liquid medium in combination with a protein matrix attached to a surface at a concentration of 0.002 to 0.15 mg/m², preferably 0.005 to 0.08 mg/m², most preferably 0.02 mg/m², The composition of the liquid medium may optionally be supplemented with the protein matrix protein at a final concentration of 40 to 1000 µg/ml, preferably 80 to 500 µg/ml, and most preferably 200 µg/ml, the preferred protein being collagen (e.g. collagen R).

The method and system, kits and cell culture medium of the present invention are useful for the production of serum-free cell cultures. These cell cultures have many uses as an *in vitro* assay system. In particular, they are suitable for evaluating gene expression. As stated above, serum supplements can seriously interfere with the results of evaluation assays for gene expression, in particular hormonally regulated gene expression and other processes such as drug metabolism processes. Therefore, this invention provides a method and cell culture system for performing such assays in vitro.

This invention can be used for producing long-term serum-free cell cultures which are stable and remain healthy over an extended period of time and are thus suitable for the evaluation of drug metabolism processes, in particular for the evaluation of liver specific drug metabolism processes if liver cells are used. The method can also be used to produce long-term differentiated cell lines. In particular, a system is provided which generates serum-free hepatocyte cultures.

Thus, the invention also provides a method of expressing recombinant gene products comprising providing cells transformed with the gene of interest and culturing said cells using the system or kit or medium according to the invention in the absence of serum or culturing the cells according to a method of the present invention.

The preferred cells to be used in these methods are hepatocyte cells, in particular the hepatocyte cell lines HuH7 and HepG2.

Another embodiment of the present invention relates to a method for promoting and/or retaining the expression of at lest four characteristics of hepatocyte differentiation selected from the expression of albumin, alpha-fetoprotein, apolipoprotein A1, cytokeratin 18, cytokeratin 19 and cytochrome P450 2C9, wherein this is achieved culturing a hepatocyte cell line according to the cell culture method of the invention or using a cell culture system according to the present invention. When hepatocyte cell lines are cultured according to the method of the present invention, they retain their differentiation characteristics for an extended period of time, in particular over a period of time of at least two months up to several months. The inventors found that if a differentiated hepatocyte cell line displays at least four of the above characteristics, it can be said to have retained hepatocyte-specific differentiation characteristics and is useful for any assays involving hepatocyte cell lines.

Thus, the present invention also provides serum-free cultured cells, in particular cell lines which are obtainable by the culture method according to the invention. Such cell lines are useful as an in vitro assay system, e.g. for evaluating gene expression, in particular hormonally regulated gene expression, and for evaluating drug metabolism, in particular liver specific drug metabolism processes.

In particular, the invention provides a differentiated hepatocyte cell line which displays at least four hepatocyte differentiation characteristics selected from the expression of albumin, alpha-fetoprotein, apolipoprotein A1, cytokeratin 18, cytokeratin 19 and cytochrome P450 2C9. Such a cell line can be used in particular for hormonally regulated gene expression *in vitro* or it can be used for evaluation of drug metabolism processes *in vitro* in particular for evaluating liver specific drug metabolism processes.

Another aspect of the present invention is a method of expressing recombinant gene products comprising providing cells transformed with a gene of interest and culturing said cells according to the cell culture method of the invention or using a system, kit or cell culture medium according to the invention.

Another aspect of the invention relates to a method for promoting the expression of at least four characteristics of hepatocyte differentiation selected from the expression of albumin, alpha-fetoprotein, apolipoprotein A1, cytokeratin 18, cytokeratin 19 and cytochrome P450 2C9, the method comprising culturing said cells according to the cell culture method of the invention or using a system, kit or cell culture medium according to the invention.
- Figure 1: shows the metabolic activity of HepG2 cells cultured on plastic dishes in the presence of serum in comparison with HepG2 cell cultured on collagen coated dishes in serum-free medium. (A) to (D) show results for cells seeded at different densities.

- Figure 2: shows the expression of serum proteins and cytokeratins in short term culture of HepG2 and HuH7 cells cultured in the presence of serum compared with the same cells cultured in serum-free media on collagen coated dishes.
- Figure 3: shows liver specific gene expression in HepG2 cells maintained under serum-free conditions for a few days (A) and for several months (B).
- Figure 4: shows expression of cytochrome P450 (CYP) proteins in human hepatoma cell lines HepG2 and HuH7 cultured in the presence of serum or in serum-free medium on collagen coated dishes.
- Figure 5: shows the induction of cytochrome P4501A protein in HuH7 and HepG2 cells cultured under chemically defined conditions.
- Figure 6: shows a two-dimensional gel analysis of HuH7 cell extracts maintained on plastic in the presence of FCS and on collagen in serum-free medium.

### Examples

Human hepatoma cells were cultured under chemically defined conditions for several months. They showed identical metabolic activities and grew at the same rate as their counterparts cultured conventionally in the presence of high serum concentrations. We demonstrate that the culture system presented here may be a useful tool to study hepatocyte specific gene expression in hepatoma cells under chemically defined conditions. It may serve as an appropriate experimental model system to evaluate hormonally regulated gene expression and liver specific drug metabolism processes in man.

### Cell culture

For this and following examples, culture flasks (75 ml) or dishes (diameter 100 mm) were coated with rat tail collagen at a concentration of 0.02 mg/cm². The collagen stock solution (2mg/ml) in 0.1% acetic acid was diluted 1:10 with H₂O. 8 ml of this dilution (total 1.6 mg) are applied to 75 -78 cm² of culture flasks or dishes (i.e. approx. 0.02 mg/cm²) and UV-cross-linked under sterile conditions overnight.

The serum-free medium used for the experiments described in the present examples is designated Human Hepatocyte Maintenance Medium (HHMM). This medium consists of 2 parts minimal essential medium with non-essential amino acids (MEM), 1 part Dulbecco's modified Eagle medium (DMEM) without glucose and 1 part of DMEM with glucose supplemented with 1 g/l albumin, 5.5 mM galactose, 0.5 mM glutamine, 0.3 mM ornithine, 0.13 mM proline, 5 mM Hepes, 2.5 ng/ml Na-selenite, 2.5 µg/ml transferrin, 0.272 mg/l ZnCl₂, 0.1 mg/l CuSO₄ x 5H₂O, 0.375 mg/l ZnSO₄ x 7 H₂O, 0.0125 mg/l MnSO₄, 2.75 µg/ml insulin, 50 nM dexamethasone, 25 µg/ml gentamycin, 100U/ml penicillin, and 100 µg/ml streptomycin.

HepG2 and HuH7 cells that were grown in 75 ml culture flasks or culture dishes (10 cm diameter) to 80-100% confluence (approx. 4.2 x 10⁶ cells) in DMEM with 10% FCS under an humidified atmosphere of 5 % CO₂, 95% air, at 37°C were separated by trypsinization as follows: the culture medium was aspirated, the cells were rinsed with 10 ml of phosphate buffered saline (PBS). The cells were incubated with 2 ml of 0.05% trypsin 0.02% EDTA in PBS (Biochrome #L2153) until all were detached from the surface of the flask. Approximately 4 ml of Human Hepatocyte Maintenance Medium (HHMM) was added to neutralise the trypsin. The so separated cells were seeded onto collagen coated flasks or plates at a density of approximately 1.4 x 10⁶ viable cells onto three 75 ml flasks or three dishes (diameter 10 cm) and the cultures were incubated at 37°C, under a humidified atmosphere in 5% CO₂. For comparison conventional cultures were run in parallel in DMEM with 10% FCS on plastic dishes or flasks. The culture medium was renewed every 2 days. Within 4-5 days the cells grew to confluence and could be harvested by trypsinization and seeded into new flasks as described above. The cellular function and polypeptide expression pattern of serum-free cultured epithelial cells was investigated by metabolic activity assays, Western blot analysis and 2 dimensional gel electrophoresis and compared to cells cultured under conventional conditions with serum on plastic surfaces.

### Example 1

### Growth rate and metabolic activity

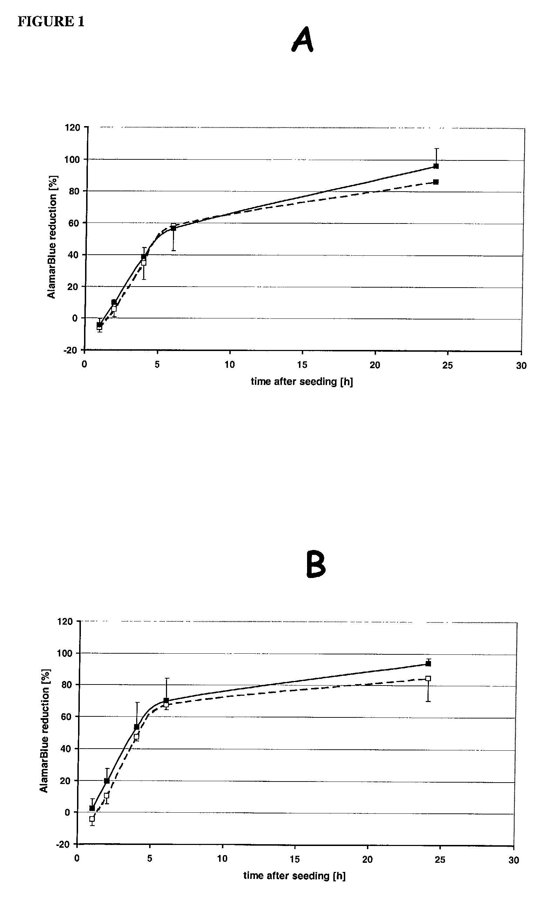

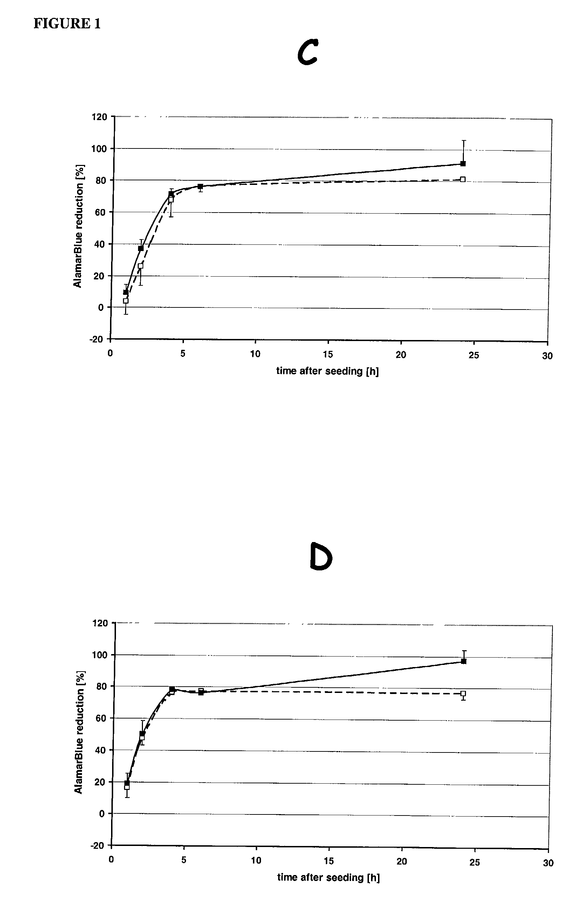

After adaptation to serum free culture conditions HuH7 and HepG2 hepatoma cells grown in chemically defined HHMM on collagen coated culture dishes exhibited identical growth rates and metabolic activities as compared to their counterparts grown under conventional conditions (Fig 1). Figure 1 shows the metabolic activity of HepG2 cells cultured on plastic dishes in the presence of serum and on collagen coated dishes in serum-free medium. HepG2 cells were seeded onto 10 cm dishes at A) approx. 7.000, B) approx. 9.300, C) approx. 14.000, and D) approx. 18.600 cells/cm² and cultured in the presence of 10% AlamarBlue™ in HHMM on collagen coated plates (filled squares, solid lines) or in DMEM with 10% FCS on plastic (open squares, broken lines). At times indicated, aliquots of culture medium were taken and optical density was measured at 570 nm (oxidised form) and 600 nm (reduced form). The percentage of reduced AlamarBlue™ substrate was calculated according to the manufacturer's protocol. Data represent mean + SD of two independent experiments. In general, the cells were passaged every 5 days. Unexpectedly, it was found that supplementing the HHMM with hepatocyte growth factor (HGF, 10 ng/ml) and epidermal growth factor (EGF, 20 ng/ml) did not improve the growth rate of hepatoma cells. On the contrary, it led to cell death after about 5-6 passages.

### Example 2

### Expression of cytokeratins

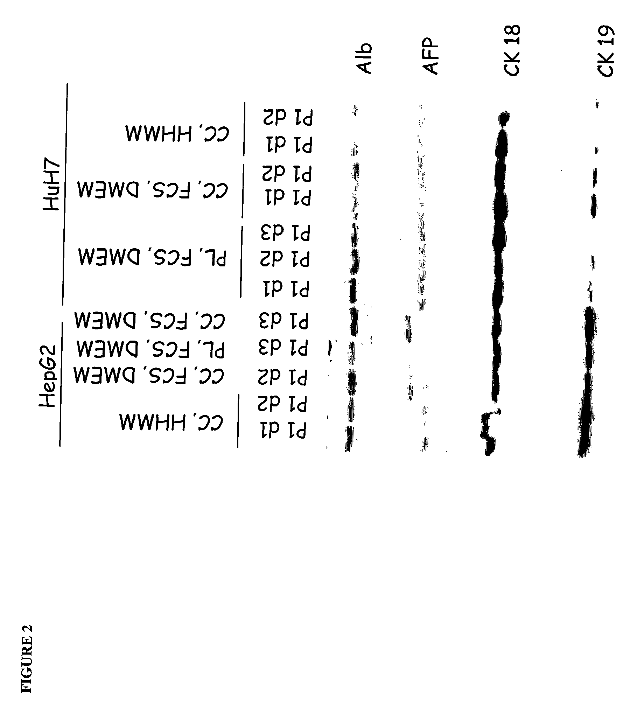

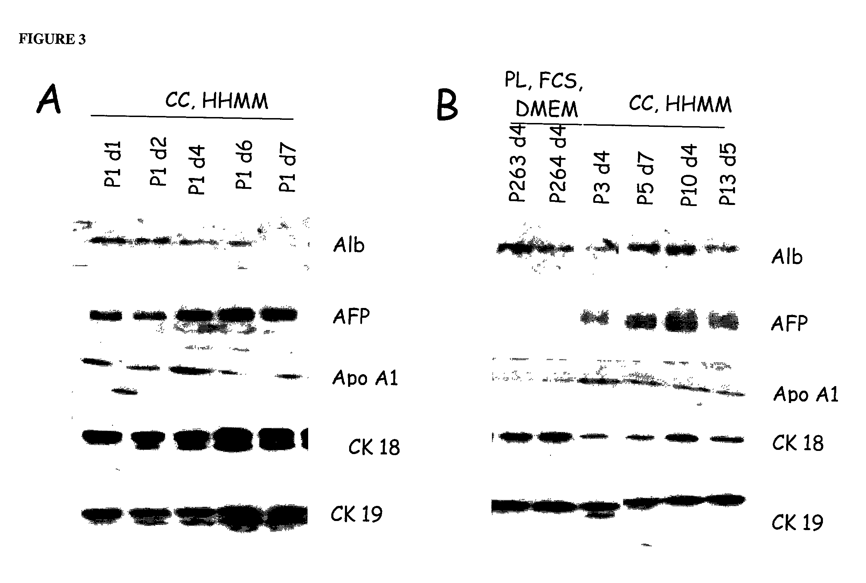

Expression of several liver specific markers was assayed. These include proteins associated with hepatocyte differentiation (albumin, apolipoprotein), and cytokeratin markers (CK18 and CK19). Both cytokeratins were expressed at high levels in HepG2, while HuH7 expressed CK19 only in small amounts (Fig. 2). Fig. 2 shows the expression of serum proteins and cytokeratins in HepG2 and HuH7 cells cultured in the presence of serum or in chemically defined media on collagen coated dishes. Hepatoma cells were seeded at a density of 14.000 cells per cm². At days indicated cells were harvested. Whole cell lysates were prepared, 40 µg protein was loaded and separated by SDS polyacrylamide electrophoreses and subjected to Western blot analysis with specific antisera as indicated. P = passage, d = day, CC = collagen coated, PL = plastic, FCS = foetal calf serum, DMEM = Dulbecco's modified Eagle medium, HHMM = Human Hepatocyte Maintenance Medium, Alb = albumin, AFP = α-fetoprotein, apoA1 = apolipoprotein A1, CK = cytokeratin
Since CK19 which is expressed specifically in bile ducts was expressed at higher levels when HHMM was used instead of DMEM (Fig. 2). Therefore, HHMM was used for further experiments. After seeding CK18 and CK19 expression increased within time as cell to cell contacts were established and the cells became more and more confluent (Fig. 3A).

### Example 3

### Expression of serum proteins

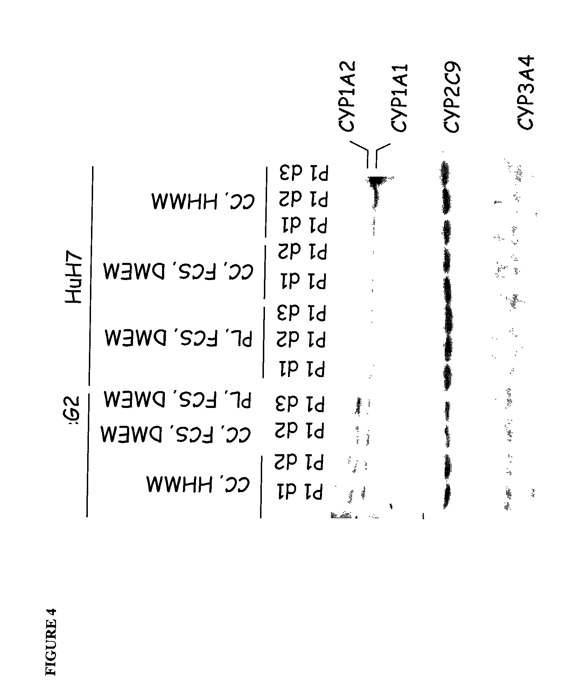

Albumin expression is transcriptionally regulated by liver specific transcription factors Hepatocyte Nuclear Factor (HNF)-1 and CCAAT/enhancer binding protein (C/EBP)-α, it is expressed in the liver and responsible for transporting fatty acids and establishing serum colloid osmotic pressure in the blood. α-fetoprotein is synthesised by foetal liver and yolk sak and expressed during foetal development. It drops to trace amounts in serum levels after birth, but high levels of α-fetoprotein are present in plasma and ascitic fluid of adults with hepatoma. Albumin and α-fetoprotein were expressed in hepatoma cells cultured under chemically defined conditions for several weeks (Fig. 3 and 4), showing that mechanisms responsible for liver specific gene transcription are maintained.

The genes for apolipoprotein A1, A-IV and CIII are organised in tandem in a multi-gene-cluster localised on the long arm of human chromosome 11. Apolipoprotein A1 is the major protein component in high density lipoproteins (HDL). HDLs are thought to play an important role in atherosclerosis prevention and longevity. Plasma HDL levels are correlated with plasma apolipoprotein A1 levels and in turn with liver apolipoprotein A1 mRNA. Apolipoprotein A1 expression is regulated by liver enriched transcription factors HNF-3 and HNF-4, retinoid X receptor-α and by apolipoprotein regulator protein-1, transcription factors that are interacting with a hepatocyte specific enhancer in the promoter region of the apolipoprotein A1 gene. Apolipoprotein A-1 was expressed throughout the culture period in hepatoma cells cultured serum-free in HHMM at higher protein level than in conventional cultures (Fig. 3B). This shows again the function of liver specific gene transcription mechanisms in our cultures and suggests that expression of hepatocyte specific transcription factors is maintained in hepatoma cells cultured under chemically defined conditions.

Fig. 3 shows liver specific gene expression in HepG2 cells maintained under chemically defined conditions for several months. HepG2 hepatoma cells were seeded at a density of approx. 18.600 cells per cm². At days indicated cells were harvested. Whole cell lysates were prepared, 40 µg protein was loaded per lane and separated by SDS polyacrylamide electrophoreses (PAGE) and subjected to Western blot analysis with specific anti-sera as indicated. P = passage, d = day, CC = collagen coated, PL = plastic, FCS = foetal calf serum, DMEM = Dulbecco's modified Eagle medium, HHMM = Human Hepatocyte Maintenance Medium, Alb = albumin, AFP = α-fetoprotein, apoA1 = apolipoprotein A1, CK = cytokeratin

### Example 4

### Expression of drug metabolising proteins

CYP1A1 and CYP1A2 proteins were expressed at low levels in HepG2, independent of the presence or absence of serum in the culture medium. In HuH7 cells only CYP1A1, but not CYP1A2 was detected by Western blot analysis. Again, the presence of serum did not result in higher protein levels (Fig. 4).

Fig. 4 shows expression of cytochrome P450 (CYP) proteins in human hepatoma cell lines HepG2 and HuH7 cultured in the presence of serum or in chemically defined medium on collagen coated dishes. Hepatoma cells were seeded at a density of 18.600 cells per cm². At days indicated cells were harvested. Whole cell lysates were prepared, 40 µg protein was loaded per lane and separated by SDS polyacrylamide electrophoreses and subjected to Western blot analysis with specific antisera as indicated. P = passage, d = day, CC = collagen coated, PL = plastic, FCS = foetal calf serum, DMEM = Dulbecco's modified Eagle medium, HHMM = Human Hepatocyte Maintenance Medium.
In humans at least five different CYP2C enzymes have been described in liver. These enzymes are involved in the metabolism of many clinical drugs. CYP2C9 is responsible for metabolism of tolbutamide, phenytoin and hexobarbital. HepG2 and HuH7 cells express high levels of CYP2C9 when cultured under serum-free conditions (Fig. 4). The CYP4A gene expression is also regulated hormonally. CYP4A1, 4A2 and 4A3 isoforms are expressed constitutively in rat liver and kidney and their expression is induced by a class of chemicals known as peroxisome proliferators, including the hypolipidemic drug clofibrate. Both hepatoma cell lines investigate here expressed CYP4A protein when cultured serum-free for several months.

### Example 5

### Induction of expression of P450

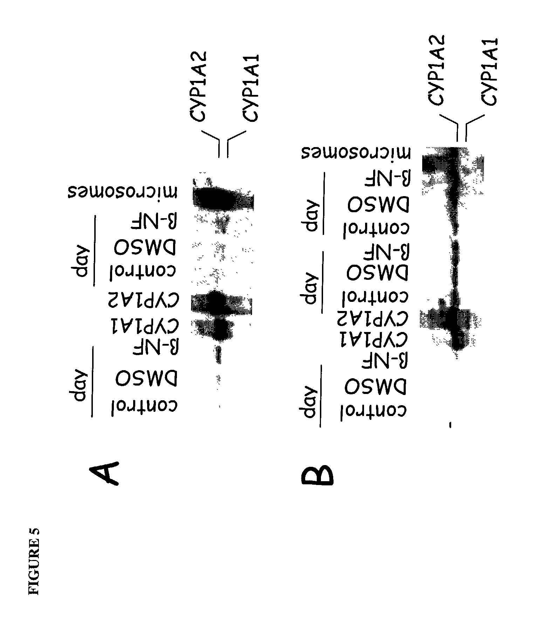

Both CYP1A1 and CYP1A2 proteins can be induced by β-naphtoflavone (β-NF), polycyclic aromatic hydrocarbon, in human hepatocyte cultures (Fig. 5).

Fig. 5 shows the induction of cytochrome P4501A protein in HuH7 and HepG2 cells cultured under serum-free conditions. HuH7 (A) and HepG2 (B) cells were seeded onto collagen coated dishes at a density of 14.000 cells per cm² and cultured in HHMM. Cells were treated with 50 µM β-NF or 0.1% DMSO for 48 h before harvested at days indicated. 40 µg of whole cell lysates were separated by SDS-PAGE, transferred onto Immobilon™ membrane and probed with specific antisera against CYP1A; CYP1A1 and CYP1A2 protein standards and/or human liver microsomes were used as controls. In non-induced HuH7 cells CYP1A levels were barely detectable.

However, treatment with 50 µM β-NF for 2 days led to an increase in CYP1A protein (Fig 5A). β-NF was also able to increase CYP1A protein levels in HepG2 cells (Fig. 5B). Other substances like DMSO had no effect on CYP1A protein levels.CYP3A4 is the predominant constitutive P450 isoform in human liver and is responsible for the metabolism of many therapeutic drugs. It is hormonally regulated: glucocorticoids and growth hormone increase 3A4 mRNA, protein and the associated testosterone-6β-hydroxylase activity while tri-iodothyronine decreases mRNA and protein levels, as well as its enzymatic activity. In our cultures CYP3A4 was expressed at low levels in HepG2 and HuH7 cells, independent of the presence of serum (Fig. 4). However, it was not possible to induce CYP3A4 protein with rifampicin, a prototypical inducer of this protein in human hepatocytes, only the addition of dexamethasone led to an increase in CYP3A4 protein (data not shown).

In primary cultures of human hepatocytes CYP1A protein expression is lost during prolonged culture, but protein inducibility by poly-cyclic aromatic hydrocarbons is maintained for several weeks (Runge et al., 2000b). In HuH7 and HepG2 hepatoma cells CYP1A protein was detectable independent of the culture conditions. Both cell lines retained the ability to increase CYP1A protein levels in response to 13-naphtoflavone, the same biological response as in human hepatocytes.

### Example 6

### 2D-gelelectrophoresis.

The cellular function and polypeptide expression pattern of serum-free cultured epithelial cells is checked by metabolic activity assays, Western blot analysis and 2 dimensional gel electrophoresis and compared to cells cultured under conventional conditions with serum on plastic surfaces.
Cells were lysed in 9M urea, 2% CHAPS and 4 mM Pefabloc SC. The lysate was incubated for 10 min at room temperature and centrifuged at 42.000 x g for 60 min. Protein concentration was determined by the BCA assay. DTT (1%) was added before isoelectric focussing. IPG strips were re-hydrated in a solution containing 8 M urea, 2% Chaps, 13 mM DTT, 0,5 % ampholytes and a trace of bromophenol blue. 80-100 µg protein was added to the rehydration solution and proteins entered the strip during re-hydration at 50 V for 14 h. Proteins were focused initially for 30 min at 500 V, then voltage was increased to 4.000 V within 1,5 h and continued for 5 h at 8.000 V for a total of 43 kVh on IPGphor (Amersham Pharmacia Biotech). For the second dimension IPG strips were equilibrated for 20 min in 50 mM Tris-HCl, pH 8.8, containing 6 M urea, 30% glycerol, 2% SDS and 65 mM DTT. Iodoacetamide (216 mM) was added to the second equilibration solution instead of DTT. The second dimension was carried out on 12% gels at 60 mA/Gel for 6 h in the Ettan-Dalt system (Amersham Pharmacia Biotech). Gels were fixed overnight in 40% ethanol, 10% acetic acid and stained with silver nitrate. The scanned gels were processed with Z3 software (Compugen, Tel Aviv, Israel).

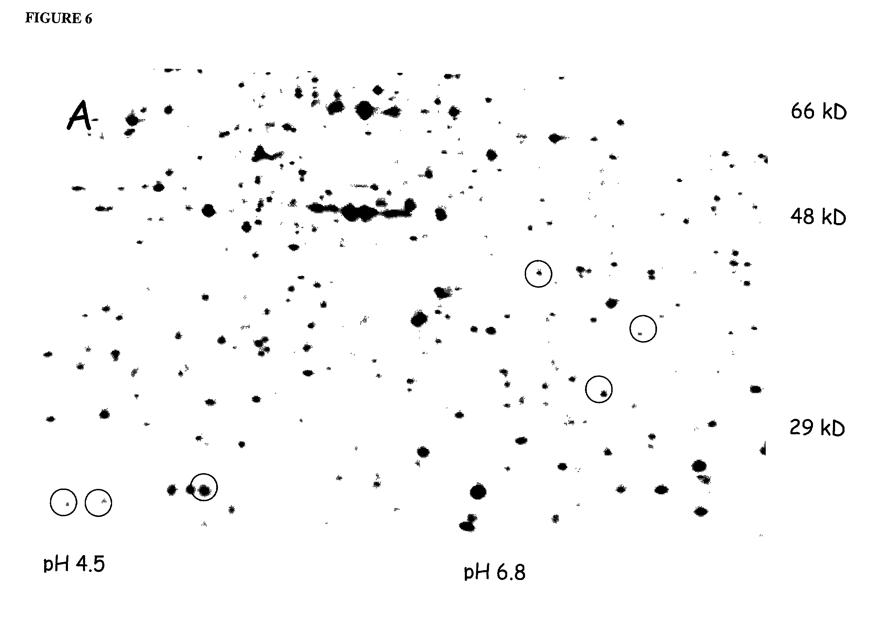

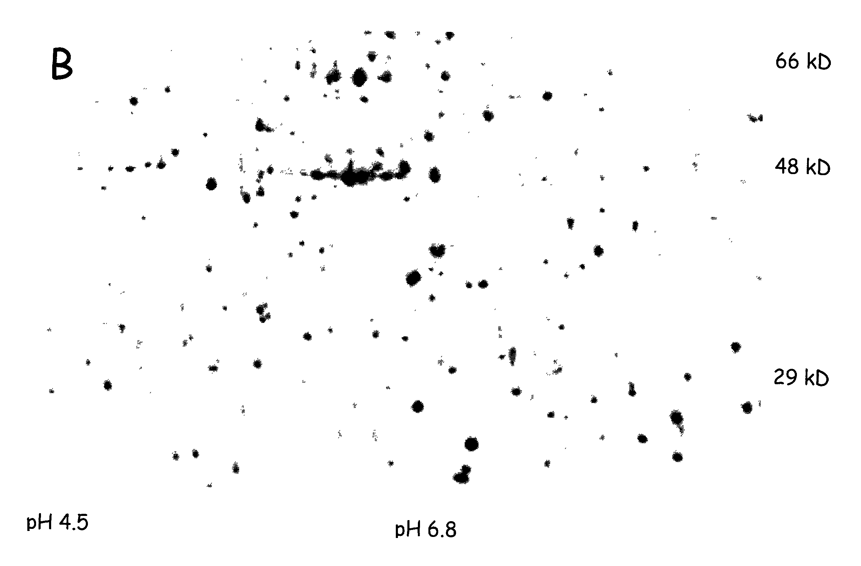

Fig. 6 shows a two-dimensional gel analysis of HuH7 cell extracts maintained on plastic in the presence of FCS and on collagen in chemically defined HHMM. HuH7 cells were seeded onto plastic and grown with 10% FCS for 6 days (A) or seeded onto collagen coated dishes and grown in HHMM for 6 days (B). Cells were harvested, proteins isolated, and separated by isoelectric focusing and subsequently by SDS-PAGE. Shown are central gel areas between pH 4.5 and 6.8 and 70 to 25 kDa. Proteins that were not expressed under serum-free conditions are circled in (A).

### References:

Moshage H, and Yap SH: Primary cultures of human hepatocytes: a unique system for studies in toxicology, virology, parasitology and liver pathophysiology in man. J Hepatol. 1992, 15(3):404-13.

Ferrini JB, Pichard L, Domergue J, Maurel P: Long-term primary cultures of adult human hepatocytes. Chem Biol Interact. 1997, 107(1-2):31-45.

Runge D, Runge DM, Jäger D, Lubecki KA, Beer Stolz D, Karathanasis S, Kietzmann T, Strom SC, Jungermann K, Fleig WE, and Micholopoulos GK: Serum-free, long-term cultures of human hepatocytes: maintenance of cell morphology, transcription factors, and liver-specific functions. Biochem Biophys Res Commun. 2000a, 269(1):46-53.

Runge D, Köhler C, Kostrtubsky VE, Jäger D, Lehmann T, Runge DM, May U, Stolz DB, Strom SC, Fleig WE, and Michalopoulos GK: Induction of cytochrome P450 (CYP)1A1, CYP1A2, and CYP3A4 but not of CYP2C9, CYP2C19, multidrug resistance (MDR-1) and multidrug resistance associated protein (MRP-1) by prototypical inducers in human hepatocytes. Biochem Biophys Res Commun. 2000, 273(1):333-41.

Aden DP, Fogel A, Plotkin S, Damianov I, Knowles BB: Controlled synthesis of HBsAg in a differentiated human liver carcinoma-derived cell line. Nature. 1979, 282(5739):615-6.

Knowles BB, Howe CC, Aden DP: Human hepatocellular carcinoma cell lines secrete the major plasma proteins and hepatitis B surface antigen. Science. 1980, 209(4455):497-9.

Adeli K and Sinkevitch C: Secretion of apolipoprotein B in serum-free cultures of human hepatoma cell line, HepG2. FEBS Lett. 1990, 263(2):345-8.

Tecce MF, and Terrana B: High-yield and high-degree purification of human alpha-fetoprotein produced by adaptation of the human hepatoma cell line HepG2 in a serum-free medium. Anal Biochem. 1988, 169(2):306-11.

Yoo BJ, Selby MJ, Choe J, Suh BS, Choi SH, Joh JS, Nuovo GJ, Lee H-S, Houghton M, and Han JH: Transfection of a differentiated human hepatoma cell line (HuH7) with in vitro-transcribed hepatitis C virus (HCV) RNA and establishment of a long-term culture persistently infected with HCV. J Virology. 1995, 69(1):32-38

Nakabayashi H, Taketa K, Miyano K, Yamane T, Sato J: Growth of human hepatoma cell lines with differentiated functions in chemically defined medium. Cancer Res. 1982, 42(9):3858-63.

Nakabayashi H, Taketa K, Yamane T, Miyazaki M, Miyano K, Sato J: Phenotypical stability of a human hepatoma cell line, HuH-7, in long-term culture with chemically defined medium. Gann. 1984, 75(2):151-8.

Michalopoulos G, Sattler GL, Pitot HC: Hormonal regulation and the effects of glucose on tyrosine aminotransferase activity in adult rat hepatocytes cultured on floating collagen membranes. Cancer Res. 1978, 38(6):1550-5.

Sattler CA, Michalopoulos G, Sattler GL, Pitot HC: Ultrastructure of adult rat hepatocytes cultured on floating collagen membranes. Cancer Res. 1978, 38(6):1539-49.

Strom SC, Michalopoulos G: Collagen as a substrate for cell growth and differentiation. Methods Enzymol. 1982, 82 Pt A:544-55.

Bissel DM, Stamatoglou SC, Nermut MV, Hughes RC: Interactions of rat hepatocytes with type IV collagen, fibronectin and laminin matrices. Distinct matrix-controlled modes of attachment and spreading. Eur J Cell Biol. 1986, 40(1):72-8.

Bissel DM, Arenson DM, Maher JJ, Roll FJ: Support of cultured hepatocytes by a laminin-rich gel. Evidence for a functionally significant subendothelial matrix in normal rat liver. J Clin Invest. 1987, 79(3):801-12

Nyberg SL, Shatford RA, Payne WD, Hu WS, Cerra FB: Primary culture of rat hepatocytes entrapped in cylindrical collagen gels: an in vitro system with application to the bioartificial liver. Rat hepatocytes cultured in cylindrical collagen gels. Cytotechnology. 1992, 10(3):205-15

Bader A, Rinkes IH, Closs EI, Ryan CM, Toner M, Cunningham JM, Tompkins RG, Yarmush ML: A stable long-term hepatocyte culture system for studies of physiologic processes: cytokine stimulation of the acute phase response in rat and human hepatocytes. Biotechnol Prog. 1992, 8(3):219-25.

Michalopoulos GK, Bowen W, Nüssler AK, Becich MJ, Howard TA: Comparative analysis of mitogenic and morphogenic effects of HGF and EGF on rat and human hepatocytes maintained in collagen gels. J Cell Physiol. 1993, 156(3):443-52.

Silva JM, Morin PE, Day SH, Kennedy BP, Payette P, Rushmore T, Yergey JA, Nicoll-Griffith DA: Refinement of an in vitro cell model for cytochrome P450 induction. Drug Metab Dispos. 1998, 26(5):490-6

Runge DM, Runge D, Dorko K, Pisarov LA, Leckel K, Kostrubsky VE, Thomas D, Strom SC, Michalopoulos GK: Epidermal growth factor- and hepatocyte growth factor-receptor activity in serum-free cultures of human hepatocytes. J Hepatol. 1999, 30(2):265-74.

### Dulbecco R , Freeman G, Virology 1959, 8:396

Eagle H, Science 1959, 130:442

Moore GE, Gerner RE and Franklin HA: Culture of normal human leukocytes. J.A.M.A. 1967, 199(8):519-524"

## Claims

1. A method for culturing cells of a cell line, comprising providing cells to be cultured, contacting said cells with a serum-free cell culture medium in a cell culture receptacle in the presence of a protein matrix, and culturing said cells in the absence of serum.

2. The method of claim 1, wherein said cells are cultured in the presence of said serum-free cell culture medium for at least 30 days.

3. The method of claim 1 or 2, wherein said cells are cultured in the presence of said serum-free cell culture medium for at least 90 days.

4. The method according to any one of claims 1 to 3, wherein said cells are cultured such that they are allowed to attach to a surface of the cell culture receptacle in the absence of serum.

5. The method according to any one of claims 1 to 4, wherein said cells are cultured under chemically defined conditions.

6. The method according to any one of claims 1 to 5, wherein said cells are vertebrate cells, in particular mammalian cells.

7. The method according to any one of claims 1 to 6, wherein said cells are human cells.

8. The method according to any one of claims 1 to 7, wherein said cells are epithelial cells.

9. The method according to any one of claims 1 to 8, wherein said cells are selected from renal cells, hepatocytes and hepatoma cells.

10. The method according to any one of claims 1 to 9, wherein said cells are differentiated cells.

11. The method according to any one of claims 1 to 10, wherein said cells are selected from the group consisting of COS cells, HuH7 cells and HepG2 cells.

12. The method according to any one of claims 1 to 11, wherein said culturing of the cells is performed in the absence of epidermal growth factor (EGF) and hepatocyte growth factor (HGF).

13. The method according to any one of claims 1 to 12, wherein the protein matrix is selected from collagen, laminin, entactin, nidogen, heparan sulphate proteoglycans, EHS gel, fibronectins and other extracellular protein matrix components or mixtures thereof.

14. The method according to any one of claims 1 to 13, wherein a surface of the cell culture receptacle is coated with the protein matrix.

15. A system for *in vitro* culturing of cells comprising cells of a cell line, a serum-free cell culture medium, a cell culture receptacle, and a protein matrix substrate.

16. The system according to claim 15, wherein the medium is not supplemented with EGF or HGF.

17. The system according to claim 15 or 16, wherein the cells have been cultured according to the method according to any one of claims 1 to 14.

18. The system according to any one of claims 15 to 17, wherein a surface of the cell culture receptacle is coated with the protein matrix substrate.

19. The system of any of claims 15 to 18, wherein the protein matrix substrate is selected from collagen, laminin, entactin, nidogen, heparan sulphate proteoglycans, EHS gel, fibronectins and other extracellular protein matrix components or mixtures thereof.

20. The system of any one of claims 15 to 19, wherein the system is a composition of matter.

21. A kit for culturing cells, comprising a serum-free cell culture medium, a cell culture receptacle, and a protein matrix substrate and optionally further cell culture supplements, wherein the protein matrix protein is dissolved or suspended in said cell culture medium.

22. A kit for culturing cells from a cell line, comprising a serum-free cell culture medium, a cell culture receptacle, and a protein matrix substrate, and optionally further cell culture supplements, wherein the cell culture medium is not supplemented with EGF or HGF.

23. The kit according to claim 22, wherein a surface of the cell culture receptacle is coated with the protein matrix substrate.

24. A serum-free cell culture medium for the serum-free culture of cells, wherein said cell culture medium comprises a protein matrix protein dissolved or suspended therein.

25. The serum-free cell culture medium according to claim 24, wherein the protein matrix protein is selected from collagen, laminin, entactin, nidogen, heparan sulphate proteoglycans, EHS gel, fibronectins and other extracellular protein matrix components or mixtures thereof.

26. The serum-free cell culture medium according to claim 24 or 25, wherein said protein matrix protein is a collagen which is present at a concentration of 0.4 to 1 mg/ml.

27. The serum-free cell culture medium according to any one of claims 24 to 26, comprising the constituents of Minimal Essential Medium (MEM) and Dulbecco's Modified Eagle Medium (DMEM) at concentrations equivalent to a combination of MEM and DMEM in a ratio ranging from 1:10 to 10:1, wherein the cell culture medium is supplemented with glutamine and optionally with further supplements selected from nutrients, amino acids, buffers, sugars, salts, trace elements, glucocorticoids, steroid hormones and differentiation factors.

28. The serum-free cell culture medium of claim 27 wherein said cell culture medium comprises the constituents of MEM and DMEM at concentrations equivalent to a combination of MEM and DMEM in a ratio ranging from 3:1 to 1:3.

29. The serum-free cell culture medium according to any of claims 27 or 28, wherein the cell culture medium is supplemented with supplements selected from albumin, proline, ornithine, transferrin, Hepes, galactose, selenite ion, Zn²⁺ ion, Cu²⁺ ion, Mn²⁺ ion, insulin, dexamethasone.

30. The serum-free cell culture medium according to any one of claims 24 to 29, wherein said cell culture medium is further supplemented with supplements selected form antibodies, antifungal and antimycotic agents, in particular gentamycin, penicillin, and streptomycin.

31. The serum-free cell culture medium according to any one of claims 24 to 30, wherein said cell culture medium is not supplemented with epidermal growth factor (EGF) or hepatocyte growth factor (HGF).

32. The method of any of claims 1 to 14, wherein said serum-free cell culture medium comprises the constituents of Minimal Essential Medium (MEM) and Dulbecco's Modified Eagle Medium (DMEM) at concentrations equivalent to a combination of MEM and DMEM in a ratio ranging from 1:10 to 10:1, and wherein said serum-free cell culture medium is supplemented with glutamine and optionally with further supplements selected from nutrients, amino acids, buffers, sugars, salts, trace elements, glucocorticoids, steroid hormones and differentiation factors.

33. The method of claim 32, wherein said serum-free cell culture medium comprises the constituents of MEM and DMEM at concentrations equivalent to a combination of MEM and DMEM in a ratio ranging from 3:1 to 1:3.

34. The method according to any one of claims 32 to 33, wherein said serum-free cell culture medium is supplemented with supplements selected from albumin, proline, ornithine, transferrin, Hepes, galactose, selenite ion, Zn²⁺ ion, Cu²⁺ ion, Mn²⁺ ion, insulin, dexamethasone.

35. The method according to any one of claims 32 to 34, wherein said serum-free cell culture medium is further supplemented with supplements selected form antibodies, antifungal and antimycotic agents, in particular gentamycin, penicillin, and streptomycin.

36. The method according to any one of claims 32 to 36, wherein said serum-free cell culture medium is not supplemented with epidermal growth factor (EGF) or hepatocyte growth factor (HGF).

37. The system of any of claims 15 to 19 or the kit of any one of claims 21 to 23, wherein said serum-free cell culture medium comprises the constituents of Minimal Essential Medium (MEM) and Dulbecco's Modified Eagle Medium (DMEM) at concentrations equivalent to a combination of MEM and DMEM in a ratio ranging from 1:10 to 10:1, and wherein said serum-free cell culture medium is supplemented with glutamine and optionally with further supplements selected from nutrients, amino acids, buffers, sugars, salts, trace elements, glucocorticoids, steroid hormones and differentiation factors.

38. The system of claim 37 or the kit of claim 37, wherein said serum-free cell culture medium comprises the constituents of MEM and DMEM at concentrations equivalent to a combination of MEM and DMEM in a ratio ranging from 3:1 to 1:3.

39. The system of claim 38 or the kit of claim 38, wherein said serum-free cell culture medium is supplemented with supplements selected from albumin, proline, ornithine, transferrin, Hepes, galactose, selenite ion, Zn²⁺ ion, Cu²⁺ ion, Mn²⁺ ion, insulin, dexamethasone.

40. The system of claim 39 or the kit of claim 39, wherein said medium is further supplemented with supplements selected form antibodies, antifungal and antimycotic agents, in particular gentamycin, penicillin, and streptomycin.

41. The system of any one of claims 37 to 40 or the kit of any one of claims 37 to 40, wherein said medium is not supplemented with epidermal growth factor (EGF) or hepatocyte growth factor (HGF).

42. Use of the serum-free cell culture medium according to any one of claims 24 to 31 for culturing cells.

43. Use of the cell culture system according to any one of claims 15 to 19, or the kit according to any one of claims 21 to 23, or the serum-free cell culture medium according to any one of claims 24 to 31, for producing serum-free cell cultures suitable for evaluating gene expression.

44. Use of the cell culture system according to any one of claims 15 to 19, or the kit according to any one of claims 21 to 23, or the serum-free cell culture medium according to any one of claims 24 to 31, for producing serum-free cultures suitable for evaluating hormonally regulated gene expression.

45. Use of the cell culture system according to any one of claims 15 to 19, or the kit according to any one of claims 21 to 23, or the serum-free cell culture medium according to any one of claims 24 to 31, for producing serum-free cultures suitable for the evaluation of drug metabolism processes.

46. Use of the cell culture system according to any one of claims 15 to 19, or the kit according to any one of claims 21 to 23, or the serum-free cell culture medium according to any one of claims 24 to 31, for producing serum-free cultures suitable for the evaluation of liver-specific drug metabolism processes.

47. A method of expressing recombinant gene products comprising providing cells transformed with a gene of interest and culturing said cells in the absence of serum:
(a) by a method according to any one of claims 1 to 14; or
(b) using a system according to any one of claims 15 to 19; or
(c) using the kit according to any one of claims 21 to 23; or
(d) using the cell culture medium according to any one of claims 24 to 31.

48. A method for promoting the expression of at least four characteristics of hepatocyte differentiation selected from the expression of albumin, alpha-foetoprotein, alipoprotein A1, cytokeratin 18, cytokeratin 19 and cytochrome P450 2C9, wherein the method comprises culturing hepatocyte cells in the absence of serum:
(a) by a method according to any one of claims 1 to 14; or
(b) using a system according to any one of claims 15 to 19; or
(c) using the kit according to any one of claims 21 to 23; or
(d) using the cell culture medium according to any one of claims 24 to 31.

49. A serum-free cell line culture obtainable by the culture method according to any one of claims 1 to 14, wherein the cells of the cell line are in the differentiated state and have remained in the differentiated state during serum-free cell culture for at least 30 days.

50. The cell line culture according to claim 49, wherein the cells of the cell line are in the differentiated state and have remained in the differentiated state during serum-free cell culture for at least 90 days.

51. The cell line culture according to any one of claims 49 or 50, wherein the cell line is a differentiated hepatocyte cell line displaying at least four hepatocyte differentiation characteristics selected from the expression of albumin, alpha-foetoprotein, apo-lipoprotein A1, cytokeratin 18, cytokeratin 19, and cytochrome P450 2C9.

52. Use of the cell line culture according to any one of claims 49 to 51 for evaluating gene expression *in vitro.*

53. Use of the cell line culture according to any one of claims 49 to 51 for evaluating hormonally regulated gene expression *in vitro.*

54. Use of the cell line culture according to any one of claims 49 to 51 for evaluating drug metabolism processes *in vitro.*

55. Use of the cell line culture according to claim 54 for evaluating liver specific drug metabolism processes *in vitro.*

56. Use of the cell culture system of any one of claims 15 to 19, or kit according to any one of claims 21 to 23, or the medium according to any one of claims 24 to 31, or the cell line culture according to any one of claims 49 to 51, for a method of producing a metabolic product of the cultured cells, wherein the method comprises partial purification of said metabolic product.

57. The use of claim 56, wherein said method provides purification or isolation of said metabolic product from other components of the cells and cell culture medium.

58. The use of any one of claims 56 to 57, wherein said metabolic product is a metabolic product of hepatocytes.

59. The use of any one of claims 56 to 58, wherein production of said metabolic product by the cultured cells is controlled by at least one recombinant gene introduced into cells of the cell line culture.

60. The use of claim 59, wherein said cells of the cell line culture act as host cells for a viral vector comprising said recombinant gene.

61. The use of any one of claims 56 to 60, wherein said metabolic product is a protein.

62. The use of any one of claims 56 to 61, wherein said metabolic product is the expression product of said recombinant gene.

63. The use of any one of claims 56 to 62, wherein said metabolic product is secreted from cells of said cell line culture into the culture medium.
